(19) Europäisches Patentamt
European Patent Office
Office européen des brevets

(11) **EP 4 793 881 A1**

(12) **EUROPEAN PATENT APPLICATION**

(43) Date of publication:
**19.08.2026 Bulletin 2026/34**

(21) Application number: **25305198.1**

(22) Date of filing: **13.02.2025**

(51) International Patent Classification (IPC):
***G06T 5/60*** *(2024.01)* ***G06T 5/70*** *(2024.01)*

(52) Cooperative Patent Classification (CPC):
**G06T 5/70; G06T 5/60;** G06T 2207/10016; G06T 2207/10132; G06T 2207/20081; G06T 2207/20084; G06T 2207/20182; G06T 2207/30048

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Dassault Systèmes**
**78140 Vélizy-Villacoublay (FR)**

(72) Inventors:
- **CHETOUI, Abdelkhalak**
  **78140 Vélizy-Villacoublay (FR)**
- **EVAIN, Ewan**
  **78140 Vélizy-Villacoublay (FR)**
- **MORALES, Hernan**
  **78140 Vélizy-Villacoublay (FR)**
- **BONNARD, Cécile**
  **78140 Vélizy-Villacoublay (FR)**
- **HERMIDA, Uxio**
  **78140 Vélizy-Villacoublay (FR)**

(74) Representative: **Bandpay & Greuter**
**11, rue Christophe Colomb**
**75008 Paris (FR)**

Remarks:
Amended claims in accordance with Rule 137(2) EPC.

(54) **GENERATION OF A SEQUENCE OF SYNTHETIC ULTRASOUND IMAGES OF AN ORGAN**

(57) A method for generation of a sequence of synthetic ultrasound images of an organ. The method comprises providing video diffusion model trained to take as input a sequence of semantically labelled 2D representations of the organ and a corresponding sequence of noise images, to generate a corresponding denoised sequence of ultrasound images respecting the labels. The method comprises providing several consecutive sequences of labelled 2D representations of the organ and corresponding consecutive sequences of noise images, iteratively applying the video diffusion model to each couple formed by one said consecutive sequence and one corresponding noise images sequence, including, at each iteration, using, for each denoising step of the backward process of the video diffusion model, a part of the denoised synthetic ultrasound images generated at the previous iteration for that denoising step in replacement of a part of images of the given couple for that denoising step.

1st 16 frames Generation
2nd 16 frames Generation
$y_0$
$y_1$
$S_0^T$
$S_0^{T-1}$
$S_0^1$
$S_0^0$
$S_1^T$
$S_1^{T-1}$
$S_1^1$
$S_1^0$

FIG. 4



Processed by Luminess, 75001 PARIS (FR)

## Description

### TECHNICAL FIELD

The disclosure relates to the field of computer programs and systems, and more specifically to a method, system and program for generation of a sequence of synthetic ultrasound images of an organ.

### BACKGROUND

Ultrasound imaging has wide use and importance in the medical context, where ultrasound images of organs are used for example to obtain medical data (*e.g.* indirect physiological measurements related to these organs). Modern tools in the medical context include software solutions for processing such images. For example, neural networks may be used to process ultrasound images of, for example, the heart, to obtain a segmentation of the heart, and/or to detect anomalies.

However, despite their increasing importance in the medical context, the availability of ultrasound imaging data remains a challenge. The availability of such data, for example in sufficient quantity to train a neural network, is often limited due to:

- Privacy concerns: strict patient privacy regulations aimed at de-identifying patient specific information.
- Operator dependency: heavy dependence on operators' expertise to obtain high-quality images and associated anatomical and functional measurements.
- Limited Standardization: Differences in equipment, settings, and protocols across institutions and vendors can lead to inconsistencies in the data collected and the resulting clinical management.
- Scarcity of cardio pediatric data: The availability of cardio pediatric data is particularly limited, making it challenging to develop comprehensive models for this patient group.

Furthermore, some organs need to be observed over time (*i.e.* a long sequence of ultrasound frames/images of the organ need to be observed over time) and there is a need to have ultrasound imaging data capturing the whole observation time needed: one image is not sufficient, a sequence of images is needed. This may be the case for organs that feature a cycle that repeats over time (the heart or the arteries for example), but also for non-cyclic organs (*e.g.* for observation of a needle insertion or of a stent/valve implantation). Neural network models, like video diffusion models, exist to generate synthetic sequences of images, or frames, to reproduce video data of a cycle. However, in the case of organ ultrasound imaging, the data at stake is computationally demanding, and due to the limitations of existing GPU, these models can only handle a limited number of images in a sequence, and that number is too much smaller than the number of frames required to model with accuracy a long sequence of frames.

Within this context, there is thus a need for improved solutions for generation of ultrasound images of organs.

### SUMMARY

It is therefore provided a computer-implemented method for generation of a sequence of synthetic ultrasound images of an organ. The method comprises providing a trained video diffusion model. The video diffusion model is trained to take as input a sequence of 2D representations of the organ and a corresponding sequence of noise images. Each 2D representation is labelled with semantic labels. The video diffusion model is trained to generate a corresponding denoised sequence of synthetic ultrasound images of the organ respecting the semantic labels. The method further comprises providing several consecutive sequences of 2D representations of the organ each labelled with semantic labels and corresponding consecutive sequences of noise images. The method further comprises iteratively applying the video diffusion model to each couple formed by one consecutive sequence of 2D representations of the organ labelled with semantic labels and one corresponding sequence of noise images. The iterative application of the video diffusion model includes, at each iteration, when applying the video diffusion model to a given couple, using, for each denoising step of the backward process of the video diffusion model, a part of the denoised synthetic ultrasound images generated at the previous iteration for that denoising step in replacement of a part of images of the given couple for that denoising step. The method may be referred to as the "noise blending method" or simply "noise blending" in the following.

The method may comprise one or more of the following:

- said part of the denoised synthetic ultrasound images generated at the previous iteration consists in ending denoised synthetic ultrasound images generated at the previous iteration and said part of images of the given couple consists in beginning images;
- a total number of images in the several consecutive sequences is larger than 40 images, and the number of images in each sequence is smaller than or equal to 24;

- the number of images in each sequence equals 16;
- the number of images in said part of the denoised synthetic ultrasound images generated at the previous iteration consists in ending denoised synthetic ultrasound images generated at the previous iteration and of images in said part of images of the given couple consists in beginning images is comprised between a quarter of and half of the number of images in each sequence; and/or
- the organ is a heart, a liver, a kidney, a carotid, an aorta, or a coronary.

It is also provided a database (*e.g.* stored on a, for example non-transitory, storage medium) comprising sequences of synthetic ultrasound images of an organ generated according to the method.
It is also provided a computer-implemented method of use of the database. The method of use comprises training a neural network based on the database. The neural network is trained for segmentation of the organ based on a sequence of ultrasound images of the organ, tracking of the organ based on a sequence of ultrasound images of the organ, detection of the organ based on a sequence of ultrasound images of the organ, registration task based on a sequence of ultrasound images of the organ, or classification task based on a sequence of ultrasound images of the organ.
It is also provided a neural network obtainable according to the method of use.
It is further provided a computer program comprising instructions for performing the method and/or the method of use.
It is further provided a device comprising a data storage medium having recorded thereon the computer program and/or the neural network and/or the database.
The device may form or serve as a transitory or non-transitory computer-readable medium, for example on a SaaS (Software as a service) or other server, or a cloud based platform, or the like. The device may alternatively comprise a processor coupled to the data storage medium. The device may thus form a computer system in whole or in part (*e.g.* the device is a subsystem of the overall system). The system may further comprise a graphical user interface coupled to the processor.

## BRIEF DESCRIPTION OF THE DRAWINGS

Non-limiting examples will now be described in reference to the accompanying drawings, where:

- FIG.s 1 to 11 illustrate the noise blending method; and

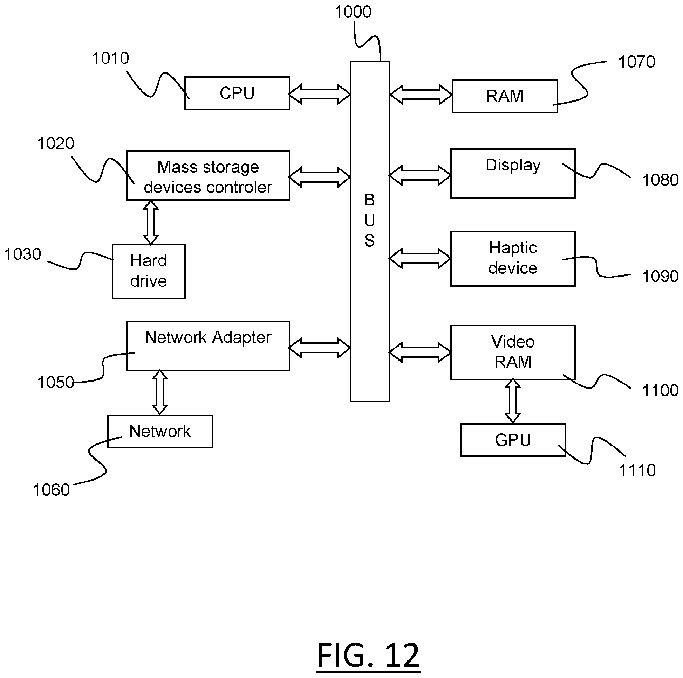

- FIG. 12 shows an example of the system.

## DETAILED DESCRIPTION

It is proposed a computer-implemented method for generation of a sequence of synthetic ultrasound images of an organ. The method comprises providing a trained video diffusion model. The video diffusion model is trained to take as input a sequence of 2D representations of the organ and a corresponding sequence of noise images. Each 2D representation is labelled with semantic labels. The video diffusion model is trained to generate a corresponding denoised sequence of synthetic ultrasound images of the organ respecting the semantic labels. The method further comprises providing several consecutive sequences of 2D representations of the organ each labelled with semantic labels and corresponding consecutive sequences of noise images. The method further comprises iteratively applying the video diffusion model to each couple formed by one consecutive sequence of 2D representations of the organ labelled with semantic labels and one corresponding sequence of noise images. The iterative application of the video diffusion model includes, at each iteration, when applying the video diffusion model to a given couple, using, for each denoising step of the backward process of the video diffusion model, a part of the denoised synthetic ultrasound images generated at the previous iteration for that denoising step in replacement of a part of images of the given couple for that denoising step.
This constitutes an improved solution for generation of synthetic ultrasound images of an organ.
Indeed, on the one hand, the method uses an already trained video diffusion model that generates synthetic ultrasound images based on noise images and semantic labels related to the organ and labelling 2D representations of that organ. Thus, the synthetic ultrasound images generated by the method have improved realism, since their generation is guided by semantic labels related to the organ at stake. On the other hand, video diffusion models are limited in terms of the number of images, or frames, that they may handle at the same time to generate a sequence. Typically, such models are trained on data of sequences of frames of an organ where each sequence comprises a small number of frames, typically 16 or sometimes 24 if very efficient hardware is available. This is due to the fact that the data at stake, i.e. video data, even if considered in images/frames forming a sequence, has high dimension and is thus computationally demanding. For this reason, existing hardware, typically existing GPU, cannot train a video diffusion model on such computationally demanding data, unless the number of frames is limited, to 16 for example or possibly 24 in rare cases. Nevertheless, for some organs, like a heart, a liver, a kidney, a carotid, an aorta, or a coronary, there is a need to generate

synthetic sequences of frames longer than 16 or 24, for example because these organs feature a cycle that repeat in over time, and one occurrence of that cycle already required more than 16 or 24 frames to be modeled, or for other long-time observations (needle insertion, or stent/valve implantation as previously explained).

To achieve this, the method considers a long sequence split in several consecutive sequences, of size processable by the video diffusion model, of labelled 2D representations of the organ and corresponding noise images and iteratively applies the trained video diffusion model to each individual sequence (of labelled 2D representations and corresponding noise images) forming the long sequence. Then, at each iteration, *i.e.* each application of the model to an individual sequence, and for that iteration, at each denoising step in the backward process of the video diffusion model, the method replaces some images (*e.g.* the first ones in the sequence) of the images of the individual sequence currently considered (*i.e.* on which the model is currently being applied) by images of the individual sequence (*e.g.* the last ones in the sequence) of images as already (partially) denoised at that denoising step at the previous iteration. To say it in other words: as known *per se* from video diffusion models, the video diffusion model takes as input a given individual sequence of 2D representations of the organ and corresponding noise images, progressively adds the noise images to the corresponding 2D representation images (forward process) and then (backward process) iteratively denoises the result, where at each iteration of the denoising (each denoising step), all images of the sequence are further denoised, until reaching a complete denoised sequence. What the method does is: in each given application of the model to each given individual sequence (*i.e.* as of the second one of course), and at each denoising step, the (partially) denoised sequence resulting from the denoising step of the backward process of the application of the model to the previous individual sequence is partially used to replace some of the images of the current sequence to denoise at that denoising step. This is referred to as "noise blending" in the present disclosure.

This noise blending allows to iteratively generate the consecutive individual sequences while keeping consistency and smooth transitions between the individual sequences, as this noise blending conditions the generation of each intermediate noisy frame of the new sequence, during the denoising (backward process), on the intermediate noisy frames of the previously generated sequence. Generating smooth and consistent sequences over time, without scene cuts, enables to reproduce synthetically the real-world clinical ultrasound acquisitions. In this context, consistency refers to preserving object appearances and background colors throughout the sequence, and disruptions are avoided. The method is thus able to generate synthetic long sequences of frames of an organ (long enough to cover a cycle of that organ) which are realistic and of quality, despite using a video diffusion model which, due to the inherent hardware limitations (*e.g.* of the existing GPUs), is limited in terms of number of frames.

The method is for generation of a sequence of synthetic ultrasound images of an organ. In other words, the method takes as input the provided several consecutive sequences of 2D representations of the organ each labelled with semantic labels and corresponding consecutive sequences of noise images, and the method then generates (through the iterative application of the video diffusion model) a corresponding long sequence of synthetic denoised ultrasound images of the organ respecting the labelling. The sequence is said to be "long" because it corresponds to the concatenation of the consecutive sequences, which may be referred to as the "individual sequences". These individual sequences are "short" in that they have a number of frames (the same for all sequences) small enough to be processed by the video diffusion model. "synthetic ultrasound image" refers to an image which is synthetic (computer-generated), and not acquired by ultrasound imaging equipment, but that reproduces as accurately as possible the features of an ultrasound image. In the present disclosure, the synthetic images are generated by a video-diffusion model (and processed by the method in the noise blending step, as explained above), so the level of accuracy with which the synthetic images resemble actual ultrasound images corresponds to the level of accuracy provided by the video diffusion model. "Denoised" means that the image is free of noise.

The method comprises providing a trained video diffusion model. The method thus provides (takes) as input an already trained video diffusion model.

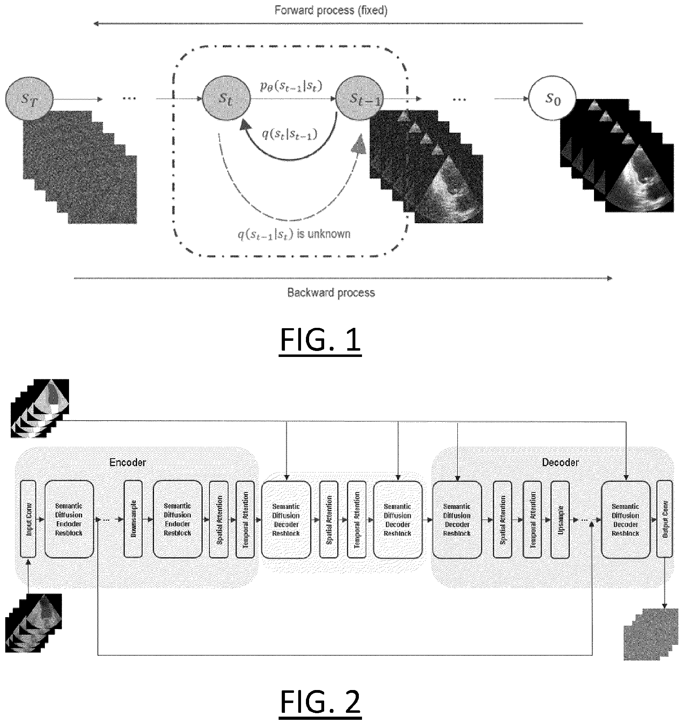

Video diffusion models are well known. A video diffusion model consists of two processes: forward and backward, as illustrated on FIG. 1. The forward process consists of adding noise progressively to the input sequence over several steps, for example T=1000 steps, until the obtention of a pure gaussian noise. The backward process consists of using a trained neural network to progressively denoise over the same number of steps, ultimately yielding a noise-free sequence.

In the present case, given a fixed number of steps T (for example T=1000) and a fixed number of frames f (for example f=16), the model does the following. It takes as input 1) a sequence of f 2D representations of the organ labelled with semantic labels, and 2) a sequence of f corresponding noise images (*i.e.* one noise image per 2D representation in the sequence). In practice the sequences may be arranged as tensors, as explained below. Then, the video diffusion model iteratively (with T iterations) denoises (backward process) the noise images with the constraint to respect the 2D representations and their semantic labels, until obtaining synthetic ultrasound images which are the result of denoising the noise images so that they become ultrasound images corresponding to the 2D representations of the organ (the "corresponding denoised sequence of synthetic ultrasound images of the organ respecting the semantic labels").

The forward process is used only during training. After that, during inference (and in particular when the model is used by the method), only the backward process is used. Specifically, during training, the model encounters training

examples each consisting of 1) a sequence of labelled 2D representations of the organ of the same type as the sequences that the model will encounter during inference/use, and 2) a corresponding sequence of real ultrasound images of the organ corresponding to the 2D representations. During training, when encountering the training examples, the model first applies a deterministic forward process which iteratively (over T steps) adds noise to the real ultrasound images given as training examples. Then, the model applies the backward process, which is a neural network that denoises the result of the forward process. The training consists in adjusting the weights/parameters of that neural network so that the denoised images correspond to the real ultrasound images in terms of texture and respect the labels of the 2D representations given as training examples.

As illustrated by FIG. 1, in implementations the model aims at maximizing the likelihood $p_\theta(s_0|y)$ given that the conditional data distribution follows $q(s_0|y)$, where $y$ denotes the sequence of ground truth labelled 2D representations and $s_0$ represents the sequence of synthetic ultrasound images to obtain. The forward process $q(s_{1:T}|s_0)$ is a process where the Gaussian noise $\varepsilon \sim \mathcal{N}(0, \mathbf{I})$ is progressively added to the ground truth images according to a variance schedule $\beta_{1,T}$:

$$q(s_t|s_{t-1}) = \mathcal{N}\left(s_t; \sqrt{1-\beta_t}s_{t-1}, \beta_t\mathbf{I}\right).$$

A high value for $T$ may be chosen such that $s_T \sim \mathcal{N}(0, \mathbf{I})$. Then, the neural network part of the model (backward process) aims at learning the reverse process:

$$p_\theta(s_{t-1}|s_t, y) = \mathcal{N}(s_{t-1}; \mu_\theta(s_t, y, t), \sum\nolimits_\theta(s_t, y, t))$$

For t=T,...,1. The gaussian noise $s_T \sim \mathcal{N}(0, \mathbf{I})$ is iteratively denoise until obtention of $s_0$ representing a valid ultrasound image (similar to the corresponding ground truth image).

Any suitable known architecture of the video diffusion model may be used. In implementations, for the neural network part (backward process), a modified version of the Semantic Diffusion Model (SDM) presented in reference Wang et al. Semantic image synthesis via diffusion models. arXiv preprint arXiv:2207.00050 (2022) (which is incorporated herein by reference) is used. Indeed, the SDM is originally conceived to image generation. However, in these implementations, its architecture is adapted for sequence generation by adding a temporal attention block after each spatial attention block as presented in reference Ho et al., Video Diffusion Models, NeurIPS 2022, which is incorporated herein by reference, and as illustrated on in FIG. 2, which illustrates the whole architecture of the model in these implementations. The temporal attention blocks are used to address the temporal consistency and long-range dependence between starting and ending frames in the 16 frames-long sequence. As shown in FIG. 2, the semantic labels of the conditioning 2D representations are injected into the decoders. This may be done using spatially-adaptive normalization ("SPADE"), which is a known method:

$$f^{i+1} = \gamma^i(y) \cdot Norm\left(f^i\right) + \beta^i(y)$$

where $f^i$ and $f^{i+1}$ are the input and output features of SPADE and *Norm* the group normalization. $\gamma^i(y)$ and $\beta^i(y)$ represent respectively the spatially-adaptive weight and bias, learnt from the semantic labels.

During inference, at each step, the noise is estimated from the input noisy sequence by the denoising network conditioned on the semantic labels. The network extracts embeddings from the semantic labels and injects them into the noisy data by applying a learnable transformation, where spatially-adaptive weights and biases are learned from the semantic layout. Using the estimated noise, a less noisy sequence is generated using a posterior probability formulation. This iterative process ensures the generation of realistic outputs faithfully guided by the semantic labels.

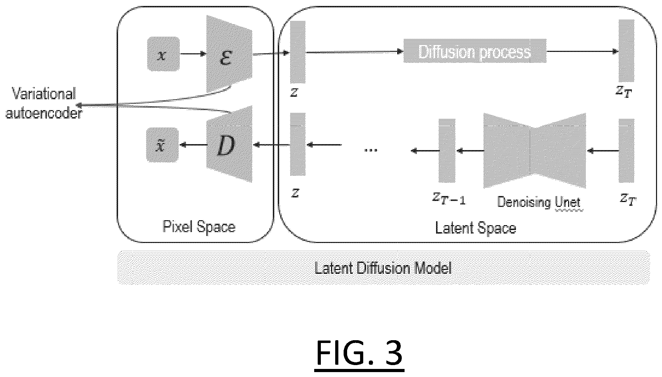

The model may process directly the pixel space of the ultrasound images (Pixel Video Diffusion Model - PVDM). Alternatively, the model may process encodings of the pixel images into a latent space after application of a previously trained variational autoencoder (Latent Video Diffusion Model - LVDM), as illustrated on FIG. 3. A pixel diffusion model is a diffusion model that operates directly in the high-dimensional pixel space of images. It involves progressively adding noise to the pixel values during the forward process and denoising them during the backward process as previously explained. While accurate, this approach is computationally intensive and memory-demanding due to the large size of pixel data. The alternative to this memory-demanding approach is the latent diffusion model. This new method is a diffusion model that operates in a lower-dimensional latent space, typically derived from a compressed representation of the data using a pre-trained encoder from a Variational AutoEncoder (VAE). By diffusing in this reduced space, latent diffusion models significantly reduce computational requirements while maintaining high fidelity, as the final image is reconstructed from the denoised latent representation using the decoder of the same VAE. For example, if the original resolution of the data is 256

$\times$ 256, the pixel diffusion model will generate images/sequences of the same resolution. However, in the latent diffusion model, the VAE will compress the data to a lower resolution, 64 $\times$ 64 in this invention. Afterwards, the diffusion process will operate in this reduced resolution and generate latent representations of 64 $\times$ 64. The VAE's decoder will map the generated latent data to a higher resolution of 256 $\times$ 256.

As previously said, the video diffusion model is provided already trained as input to the method. Providing the video diffusion model may thus comprise retrieving/obtaining the model from a memory or server or database (e.g. a remote one) where it has been stored further to its training. Alternatively, the method may comprise training the model, by any suitable known training method.

In the present disclosure, a 2D representation of the organ is a 2D image that represents the geometry of a 2D view of the organ, such as a sectional view of the organ (for example a 2D sectional view of the left atrium and ventricle of the heart). The 2D view is the same for all representations involved in the method. The 2D view may correspond to the view that would be captured by ultrasound imaging but only represents the corresponding geometry of the organ. In other words, it represents a geometry corresponding to what would be seen with ultrasound imaging, but without the texture that ultrasound imaging had. The 2D image may stem from a mesh representation of the organ and may be obtained therefrom by any suitable method. The 2D image may consist of a collection of 2D pixels altogether representing the geometry of the organ from the viewpoint of that 2D view. The 2D representation may be labelled as follows: each pixel is labeled with a semantic label of a predefined list, the predefined list consisting of: a background label (indicating absence of organ), and one label per zone of interest of the organ. Thus, each pixel is labelled with a label that indicates if it does not represent a part of the organ, or if it does and which part it represents. The 2D representations may for that reason also be referred to as semantic label maps. For example, if the organ is the heart, the labels may be "background", "left ventricle blood pool", "left ventricle myocardium", "atrium blood pool" (in applications where only the left ventricle and atrium are of interest). Alternatively, the label background may equivalently be replaced by the absence of label. But these are only examples: any meaningful semantic labeling of the organ can be used. For a given organ, the same set of semantic labels is considered for all sequences then involved in the method and in the training of the video diffusion model.

In the present disclosure, the 2D representations are always considered in sequence of N representations (N being always the same for all sequences in the method). "Sequence" means that the N 2D representations correspond to a coherent time evolution of the organ (for example the sequence represents a portion of a cycle of the organ), i.e. the first frame of the sequence corresponds to the organ at a certain time point, the second frame corresponds to the organ at that time point plus a time step, the third frame corresponds at the organ in the time point plus two time steps, and so on.

Any sequence of noise image herein always corresponds to a sequence of 2D representations in that there are as many frames/images in the noise image sequence as there are in the sequence of 2D representations. What are the noise images and how they differ from each other does not matter, as the point is to denoise each of them with the conditioning of the sequence of 2D representations so that the noises images become corresponding ultrasound images of the organ respecting the labels. It may however be required that any sequence of noise images herein correspond (*e.g.* be identical) to the sequence obtained by the video diffusion model after its forward process: if the forward process results in a sequence of noise image corresponding to a gaussian noise, any sequence of noise images herein must equally correspond to a gaussian noise. For example, any noise images sequence herein may be formed by a gaussian noise tensor. The gaussian noise tensor may be of shape $c \times f \times h \times w$, where *c, f, h and w* are the number of channels, the number of frames, the height and width respectively. As previously explained, when applying the video diffusion model (or rather its backward process), this tensor is iteratively denoised until obtention of a sequence of the same shape representing the sequence of the organ that corresponds to the corresponding sequence of 2D representations. The number of denoising steps used during the inference are *s* steps. If all the tensors acquired during the denoising process are grouped, a tensor of shape s times the initial tensor shape, i.e., $s \times c \times f \times h \times w$ is obtained.

The method also comprises providing (*i.e.* as input) several consecutive sequences (*i.e.* individual sequences) of 2D representations of the organ each labelled with semantic labels (*i.e.* each individual sequence is of the same type as the ones that the model is trained to take as input) and corresponding sequences of noise images (*i.e.* one sequence of noise images per individual sequence). "consecutive" means that the sequences altogether follow one another so as to represent a consistent time evolution (with one image/frame representing an increment of a certain time step) of the organ. The consecutive sequences represent for example altogether a cycle of the organ. Providing the consecutive sequences may for example comprise providing a single long sequence of 2D representations (and corresponding noise images) which corresponds to or at least comprises a time period equal to the time length of a cycle of the organ, and splitting it into the individual sequences. The long sequence may be obtained from a relevant dataset (like the dataset discussed after).

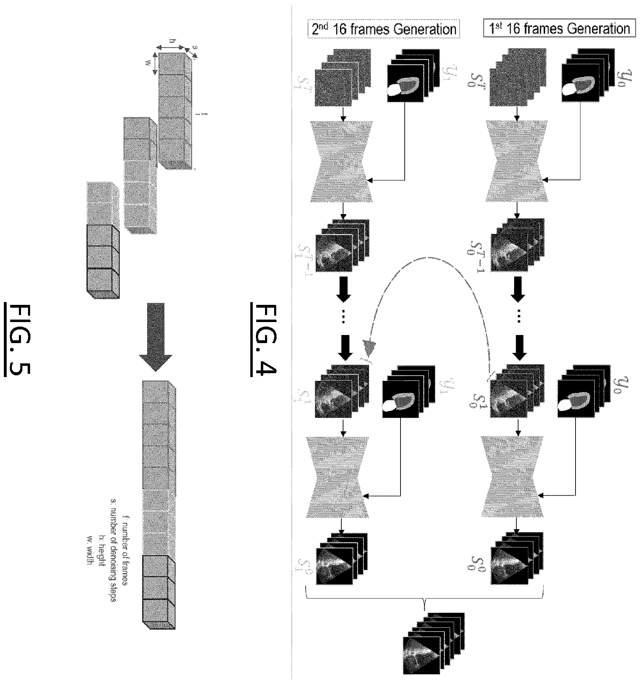

Further to the providing of the inputs, the method further comprises iteratively applying the video diffusion model to each couple formed by one of the provided individual sequences of 2D representations and its one corresponding noise images sequence. This iterative application of the model includes, at each iteration, when applying the video diffusion model to a given couple, using, for each denoising step of the backward process of the video diffusion model, a part of the denoised synthetic ultrasound images generated at the previous iteration for that denoising step in replacement of a part of images of the given couple for that denoising step. In other words. Said part of the denoised synthetic ultrasound images

generated at the previous iteration may consist in ending denoised synthetic ultrasound images generated at the previous iteration and said part of images of the given couple may consist in beginning images. This replacement in the iterations is now explained with reference to FIG. 4.

At the first iteration, the first individual sequence in the set of consecutive sequences is considered. This first individual sequence consists in the sequence $y_0$ of f labelled 2D representations of the organ (f=16 in the example illustrated on FIG. 4) and the corresponding sequence $S_0^T$ of noise images. The first iteration then consists in applying the backward process of the video diffusion model to progressively denoise $S_0^T$ into a corresponding sequence of ultrasound images $S_0^0$, , with the conditioning of $y_0$ so that $S_0^0$ respects the labelling. $S_0^T, S_0^{T-1}, \ldots, S_0^1, S_0^0$ represent all the intermediary results iteratively obtained at each denoising step. Thus, the first iteration consists solely in applying the model to the first sequence, *i.e.* there is no frame replacement ("noise blending") at this point.

At the second iteration, the second individual sequence in the set of consecutive sequences is considered. This second individual sequence consists in the sequence $y_1$ of the next f labelled 2D representations of the organ (still f=16 in the example illustrated on FIG. 4) and the corresponding sequence $S_1^T$ of next noise images. The second iteration then consists in applying the backward process of the video diffusion model to progressively denoise $S_1^T$ into a corresponding sequence of ultrasound images $S_1^0$, with the conditioning of $y_1$ so that $S_1^0$ respects the labelling. $S_1^T, S_1^{T-1}, \ldots, S_1^1, S_1^0$ represent all the intermediary results iteratively obtained at each denoising step. As illustrated by FIG. 4, as of this second iteration, frame replacement is performed: at each denoising step to obtain $S_1^{i-1}$ from $S_1^i$ (for each i between Tand 1), a given part of the frames of $S_1^i$ (the first/beginning half in the figure, but alternative choices may be considered) are replaced by a part of the frames of $S_0^i$ (the last/ending half in the figure, but again alternative choices may be considered), and then a further denoising step is performed on this sequence $S_1^i$ with the given part of its frame crushed and replaced by the part of the frames of $S_0^i$, to obtain $S_1^{i-1}$.

Although not illustrated on FIG. 4, the same is done for the next individual sequence $S_2^T$: at each denoising step to obtain $S_2^{i-1}$ from $S_2^i$ (for each i between Tand 1), a part of the frames of $S_2^i$ (beginning half for example) are replaced by a part of the frames of $S_1^i$ (ending half for example), and then a further denoising step is performed on this sequence $S_2^i$ with a part of its frame crushed and replaced by a part of the frames of $S_1^i$, to obtain $S_2^{i-1}$. And so on for the next individual sequences $S_3^T, S_4^T, \ldots S_M^T$, until the video diffusion process has been applied to all the consecutive sequences $S_0^T, S_1^T, \ldots S_M^T$.

Said part of the denoised synthetic ultrasound images generated at the previous iteration may consist in the ending denoised synthetic ultrasound images generated at the previous iteration and said part of images of the given couple may consist in the beginning images. In other words, to continue the explanation given above with reference to FIG. 4, for any m between 1 and M and i between Tand 1, at each denoising step to obtain $S_m^{i-1}$ from $S_m^i$, the beginning images of $S_m^i$ are replaced by the ending images of $S_{m-1}^i$. For example, the n beginning images of $S_m^i$ are replaced by the n ending images of $S_{m-1}^i$, with n being for example comprised between f/4 and f/2.

The total number of images in the several consecutive sequences (i.e. the total number of images in the whole long sequence $S_0^T, S_1^T, \ldots S_M^T$) may be larger than 40 images. In other words, the total number of 2D representations in the several consecutive sequences is larger than 40, and the total number of noise images is also larger than 40 since it equals the total number of 2D representations. The number of images (i.e. the number of 2D representations, which equals the number of noise images) in each individual sequence $S_m^T$ may be smaller than or equal to 24, for example equal to 16.

It is to be understood that the total number of 2D representations/noise images is not necessarily a multiple of 16. If this is not the case, for example if the total number equals 73 (which is just an illustrating example), the method may proceed as follows:

- Frames from 1 to 16 are generated, then
- Frames 13 to 28 are generated, then an overlap of 4 frames is made (13-16),
- Frames from 25 to 40 are generated, an overlap of 4 frames is made (25-28), then,
- Frames from 37 to 52 are generated, an overlap of 4 frames is made (37-40), then
- Frames from 49 to 64 are generated, an overlap of 4 frames is made (49-52), then
- Frames from 58 to 73 are generated, an overlap of 7 frames is made (58-64).

The above example of overlap is not unique and may be adapted to any total number of frames, as long as the overlap step is carefully chosen to keep all the frames. In the example the overlap step is chosen in the interval [16/4=4, 16/2=8]. The overlap step may be selected by the user (*e.g.* at an initial step of the method) or may be selected automatically so as to respect the above rule to keep all the frames.

The frame replacement (noise blending) is further illustrated by FIG. 5. As can be seen from FIG. 5, to generate a long ultrasound sequence while using a video diffusion model trained to generate only $f$ = 16 frames at a time, a first sequence of shape $c \times f \times h \times w$ is generated. All the s intermediate tensors produced during the denoising process are saved. The next step consists of extending the length of the current first sequence. For this purpose, an overlapping is used. Indeed, during the generation of the new sequence, its intermediate noisy tensors of the first $n \in \left[0, \frac{f}{2}\right]$ frames will be replaced by the intermediate noisy tensors of the last $n$ frames of the previously generated sequence. Also, the first $n$ frames of the newly generated sequence will have the same semantic labels conditioning as the last $n$ frames of the previously generated sequence. As a sum, only 16 - $n$ new frames will be generated each time. They will be directly concatenated to the resulting sequence of previous generations.

Evaluations of the noise blending proposed in the present disclosure are now discussed. For these evaluations, the inventors have trained the video diffusion model with a dataset for 2D echocardiographic assessment. The dataset contains 2D apical four-chamber and two-chamber views at end diastole and end systole, with sequences of a half cardiac cycle acquired from 500 patients. Each acquisition is accompanied by corresponding segmentations of the left ventricle myocardium, left ventricle blood pool, and left atrium. Since the right ventricle and right atrium are not segmented in the four-chamber view, it was decided to train the model only on two-chamber views.

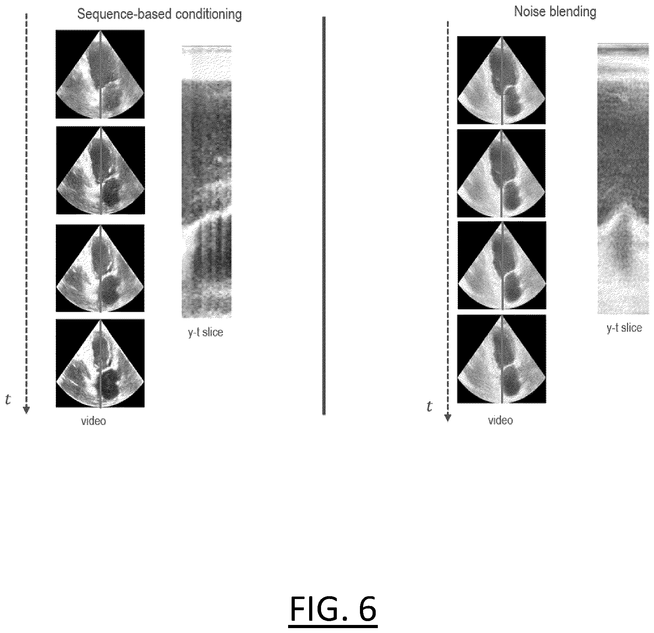

First the inventors compared the noise blending method with a sequence-based conditioning method. In the latter, the new sequence is conditioned directly on the previously generated one, rather than on each of its noisy intermediate frames. FIG. 6 illustrates the comparison. The inventors compared the y-t slice of a synthetic ultrasound of a specific patient generated by both methods, where the y-t slice is formed by extracting the same vertical line from each frame in the sequence and stacking these lines horizontally to create a single image. The sequence-based conditioning method shows noticeable scene cuts between each chunk generation, while the noise blending method produces seamless and smooth transitions.

The inventors then compared the use of noise blending method with a pixel diffusion model and with a latent diffusion model.

In order to evaluate the noise blending method applied with pixel and latent diffusion models, they generated two datasets of 200 long cardiac ultrasound sequences with both methods, all comprising a whole cardiac cycle. The number of frames, depending on each patient from the dataset, varies from 40 to more than 80 frames.

The intention of the evaluation is to assess the quality of the invention on two main points:

- Temporal consistency despite the generation of the long sequence being done over multiple batches.
- Fidelity of the generated sequence to the semantic labels used for guidance.

Generally, generative artificial intelligence methods lack rigorous metrics to evaluate the quality of generated sequences and images. In the following, all the metrics deemed interesting are discussed.

**Temporal consistency:**

Regarding the first evaluation point, the y-t slice of the noise blending method shows better temporal consistency in comparison to the sequence-based conditioning method (see again FIG. 6). In addition to the y-t slice, the Fréchet video Distance (FVD) and Fréchet Inception Distance (FID) metrics are employed. Both metrics measure the Fréchet distance between the real-world data distribution and the distribution defined by the generative model. FVD is designed for video datasets, while FID is tailored for image datasets. Specifically, both metrics utilize pretrained networks -one on videos and the other on images- to extract features from real-world and synthetic datasets, enabling meaningful comparison. In this work, FVD is used to compare sequences and FID is applied to compare individual images, which in this case correspond to the frames of the sequences. For both metrics, the lower the value the better the performance of the method.

Table 1: comparison between datasets generated by PVDM and LVDM

| | FID ↓ | FVD ↓ |
|---|---|---|
| Pixel Video Diffusion Model (PVDM) | 131.66 | 396.42 |
| Latent Video Diffusion Model (LVDM) | 126.14 | 540.06 |

LVDM's lower FID value indicates that its frame-by-frame images are closer to the original database distribution. Conversely, PVDM's lower FVD value suggests better temporal consistency.

**Labels fidelity:**

To assess the fidelity of synthetic sequences to input labels, a segmentation neural network is trained on real-world ultrasound sequences and applied on synthetic sequences. The segmentation results obtained by this neural network are then compared with the input labels. The higher the scores, the better the fidelity. The network used for this task is the nnU-Net discussed in Isensee,et al. (2018). nnu-net: Self-adapting framework for u-net-based medical image segmentation. arXiv preprint arXiv:1809.10486, which is incorporated herein by reference. This neural network is recognized as the one of the best segmentation networks for medical imaging.

In Table 2, four metrics are used to assess the quality of the segmentation:

- Dice score: $\frac{2\times Area\ of\ overlap\ of\ ground\ truth\ and\ prediction}{total\ \ area{:}ground\ truth+prediction}$
- Intersection over Union (IoU): $\frac{Area\ of\ overlap\ of\ ground\ truth\ and\ prediction}{Area\ of\ union\ of\ ground\ truth\ and\ prediction}$
- Precision: $\frac{true\ positive}{true\ positive+false\ positive}$
- Recall: $\frac{true\ positive}{true\ positive+false\ negativae}$

| | Dice Score | | | | IoU | | | | Precision | | | | Recall | | | |
|---|---|---|---|---|---|---|---|---|---|---|---|---|---|---|---|---|
| | 0 | 1 | 2 | Avg | 0 | 1 | 2 | Avg | 0 | 1 | 2 | Avg | 0 | 1 | 2 | Avg |
| Pixel diffusion dataset | 0.9450 | 0.808 | 0.937 | 0.897 | 0.898 | 0.682 | 0.888 | 0.822 | 0.960 | 0.724 | 0.927 | 0.870 | 0.934 | 0.923 | 0.954 | 0.937 |
| Latent diffusion dataset | 0.912 | 0.724 | 0.906 | 0.847 | 0.842 | 0.573 | 0.834 | 0.749 | 0.943 | 0.601 | 0.911 | 0.818 | 0.890 | 0.925 | 0.909 | 0.908 |

Table 2: segmentation metrics on datasets generated by Pixel and latent video diffusion models.

The semantic labels consist of 3 classes: 0 for the left ventricle blood pool, 1 for the left ventricle myocardium and 2 for the atrium blood pool. Table 2 also includes an average value calculated across the three classes.

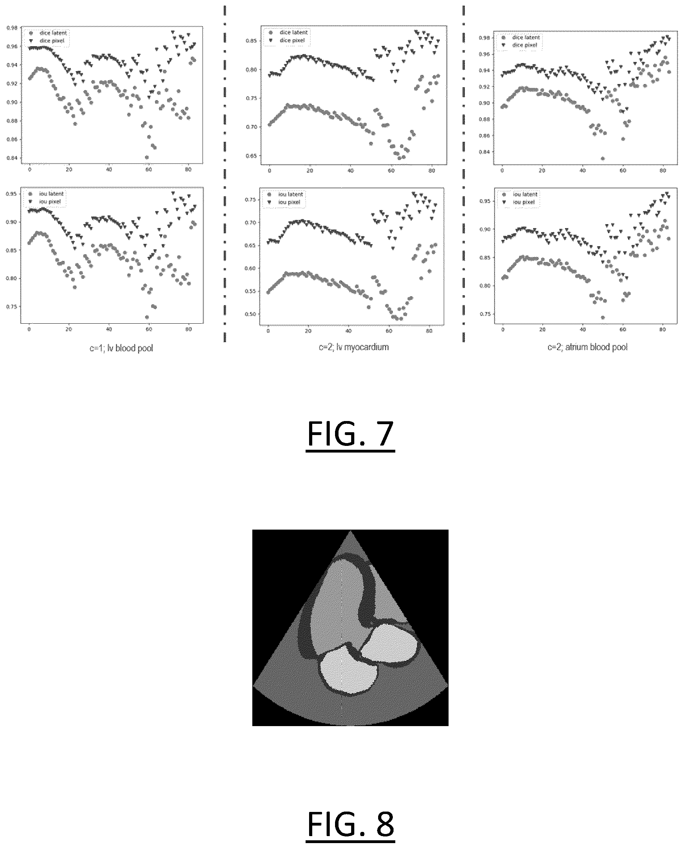

The numerical results in Table 2 indicate that PVDM more accurately follows the original semantic labels during generation. Indeed, nearly all the scores achieved on the PVDM dataset are higher. FIG. 7 reports the Dice score and IoU on a frame-by-frame basis. PVDM achieves higher scores not only on average but also consistently across all individual frames.

So far, the results have shown that the PVDM exhibits better temporal consistency and fidelity to semantic labels.

As an additional evaluation to assess the generalizability of the pixel and latent diffusion models, an attempt is made to generate images of the apical three-chamber view, despite the model being trained exclusively on two-chamber views. It is noted that sequences of semantic labels for the apical three-chamber view are unavailable, which is why only the image generation is attempted to assess how the pixel and latent diffusion models generalize to unseen views. FIG. 8 shows an apical three chamber 2D representation with semantic labels. FIG. 9 and FIG. 10 show two apical three-chamber views generated by the pixel and latent diffusion models.

Visually, the pixel diffusion model shows its ability to generate unseen cardiac views, while the latent diffusion model struggles to generate the aorta. This can be attributed to the fact that the aorta was not included during training. This demonstrates that the pixel diffusion model is able to generalize effectively, while the latent diffusion model is not.

More rigorously, two datasets of apical three chamber views are generated with both methods. Morphological

snakes segmentation (discussed in reference Marquez-Neila et al. (2013). A morphological approach to curvature-based evolution of curves and surfaces. IEEE Transactions on Pattern Analysis and Machine Intelligence, 36(1), 2-17, which is incorporated herein by reference), an unsupervised segmentation method, is used to detect the three chambers. The goal is to determine whether the aorta will be detected, serving as an indication of the generative model's generalizability.

| a3ch | Dice Score | | | IoU | | | Precision | | | Recall | | |
|---|---|---|---|---|---|---|---|---|---|---|---|---|
| | Left ventricle bp | Left atrium bp | aorta bp | Left ventricle bp | Left atrium bp | aorta bp | Left ventricle bp | Left atrium bp | aorta bp | Left ventricle bp | Left atrium bp | aorta bp |
| Pixel diffusion dataset | **0.692** | 0.724 | 0.696 | **0.532** | 0.570 | 0.536 | **0.996** | **0.993** | 0.995 | **0.533** | 0.573 | 0.538 |
| Latent diffusion dataset | 0.638 | **0.736** | 0.318 | 0.474 | **0.593** | 0.203 | 0.949 | 0.821 | 0.614 | 0.488 | **0.694** | 0.292 |

Table 3: Apical three chamber view segmentation scores.

The metrics indicate that the pixel space diffusion model can generalize to previously unseen organs (e.g., aorta). The latent diffusion models achieved competitive scores only for the left ventricle and left atrium, as these were part of the training dataset. However, the performance metrics for the aorta were notably poor for the latent diffusion model.

**Conclusion:**

This noise blending method has demonstrated its ability to generate long cardiac ultrasound sequences guided by semantic labels. As previously stated, noise blending allows the generation of cardiac ultrasound videos with the following two conditions grouped together:

- Long cardiac ultrasound videos with arbitrary number of frames using diffusion models.

- Guided sequences by semantic labels.

The evaluations illustrate the main advantage of the proposed method: to generate long videos of cardiac ultrasound guided by semantic labels with arbitrary number of frames. Generally, as previously mentioned, video diffusion models are trained on a small number of frames, due to computing power limitation. With noise blending, it is possible to generate a geometry-guided ultrasound videos regardless of how long the cardiac cycle lasts for each patient.

The advantages illustrated by the above evaluations can be grouped in the list below:

- Generation of long-duration videos: capable of creating ultrasound videos of arbitrary lengths while maintaining consistent quality throughout the entire duration, without degradation.
- Adaptability to individual cardiac cycles: customizes video generation based on each patient's unique cardiac cycle duration.
- Overcoming computational constraints: the generation of long videos cardiac ultrasound no longer requires the model to be trained on a high number of frames. For instance, training a model to generate 16 frames is enough to generate videos of more than 60 frames in inference.
- Geometry-guided generation: Uses semantic labels to guide the video generation, ensuring that the anatomical details and movement of the heart are realistic and clinically accurate.

The evaluations of the proposed noise blending method have been performed in the context of heart images (*i.e.* the organ in the method is a heart). However, as previously explained, the method is not limited to that specific organ. For example, the organ may be heart, a liver, a kidney, a carotid, an aorta, or a coronary. The method may generally be used to generate ultrasound sequences of any organ from labels where the sequence of ultrasound images is needed for diagnostic, therapeutic planning, during intervention and follow up. The presented evaluation tests were performed on cardiac use case (see for example reference Ohte, N., Ishizu, T., Izumi, C., Itoh, H., Iwanaga, S., Okura, H., ... & Japanese Circulation Society Joint Working Group. (2022). JCS 2021 guideline on the clinical application of echocardiography. Circulation Journal, 86(12), 2045-2119, which is incorporated herein by reference, for a discussion on this specific application case) but the method applies to other organs/arteries that may involve ultrasound sequences, such as carotid (see Townsend, R. R., Wilkinson, I. B., Schiffrin, E. L., Avolio, A. P., Chirinos, J. A., Cockcroft, J. R., ... & Weber, T. (2015). Recommendations for improving and standardizing vascular research on arterial stiffness: a scientific statement from the American Heart Association. Hypertension, 66(3), 698-722, which is incorporated herein by reference), aorta (see Writing

Committee Members, Isselbacher, E. M., Preventza, O., Hamilton Black III, J., Augoustides, J. G., Beck, A. W., ... & Woo, Y. J. (2022). 2022 ACC/AHA guideline for the diagnosis and management of aortic disease: a report of the American Heart Association/American College of Cardiology Joint Committee on Clinical Practice Guidelines. Journal of the American College of Cardiology, 80(24), e223-e393, which is incorporated herein by refernce), liver (see Kelly, E. M., Feldstein, V. A., Parks, M., Hudock, R., Etheridge, D., & Peters, M. G. (2018). An assessment of the clinical accuracy of ultrasound in diagnosing cirrhosis in the absence of portal hypertension. Gastroenterology & hepatology, 14(6), 367, which is incorporated herein by reference), kidney (see Hansen, K. L., Nielsen, M. B., & Ewertsen, C. (2015). Ultrasonography of the kidney: a pictorial review. Diagnostics, 6(1), 2, which is incorporated herein by reference), coronary (see Xia, M.; Yan, W.; Huang, Y.; Guo, Y.; Zhou, G.; Wang, Y. IVUS Image Segmentation Using Superpixel-Wise Fuzzy Clustering and Level Set Evolution. Appl. Sci. 2019, 9, 4967. https://doi.org/10.3390/app9224967, which is incorporated herein by reference). Adapting the training database to the use case and taking into account the acquisition frequency and the probe, allows to generate label-accurate data using the method presented in this disclosure.

For example, on the carotid, the motion extracted from the carotid artery wall provides unique information for vascular heart evaluation. As explained in reference Rizi, F. Y., Au, J., Yli-Ollila, H., Golemati, S., Makunaité, M., Orkisz, M., ... & Zahnd, G. (2020). Carotid wall longitudinal motion in ultrasound imaging: an expert consensus review. Ultrasound in Medicine & Biology, 46(10), 2605-2624, which is incorporated herein by reference, the longitudinal wall motion corresponds to the displacement of tissue layers in the direction parallel to the blood flow during the cardiac cycle. To generate relevant data to tackle this problem, the associated labels could be the lumen, intima, the media and the adventitia of the carotid and the atherosclerosis plaque.

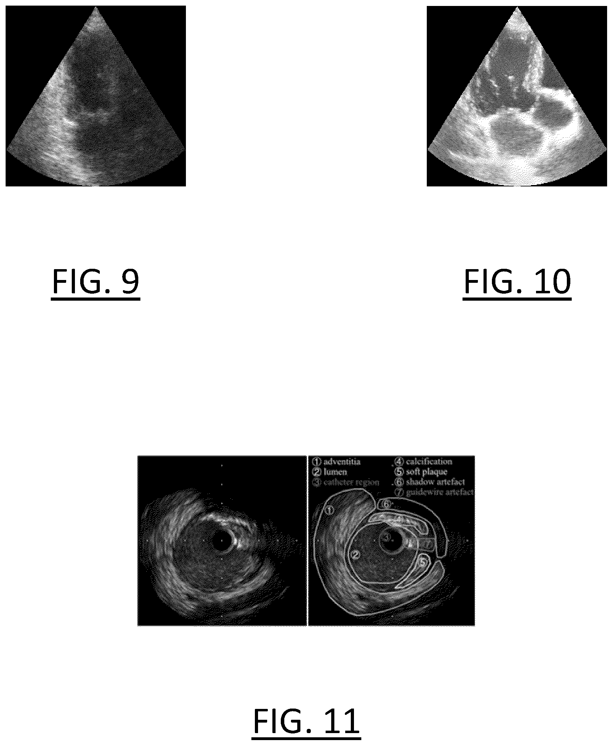

Likewise, intravascular ultrasound (IVUS) sequences, as shown in FIG. 11, are acquired for coronary arteries to characterize and measure calcifications. Segmentation of structures, classification and tracking are required to identify where the calcifications are and how do they relatively move with respect to the artery, which can be later used to assess a plaque rupture risk.

The study of the liver is a huge challenge due to the complexity of the organ (structure, vascularization) and the motion induced by the breathing. This motion often deforms the structures of interest such as cysts. The proposed method can be used to generate a synthetic ultrasound database taking into account the beathing cycle to induce motion in the simulated data and therefore handle various physiological shapes. In this example, the labels may be different kind of cysts, vessels, liver tissue.

It is also proposed a database comprising sequences of synthetic ultrasound images of an organ generated according to the method. In other words, this database consists of data pieces, each piece being a long synthetic sequence of ultrasound images of an organ resulting from the step of iterative application of the video diffusion model. There may be one such database per organ that can be considered (e.g. heart, liver, kidney, carotid, aorta, coronary). The database (or each database, one per organ) may be stored on a transitory or non-transitory computer-readable storage medium.

It is also proposed a computer-implemented method of use of the database. The method of use may be performed independently from the noise blending method previously discussed. Alternatively, both methods may be part of the same computer-implemented process. The method of use comprises training a neural network based on the database, the neural network being trained for one of the following tasks:

- segmentation of the organ based on a sequence of ultrasound images of the organ, *i.e.* the neural network is trained to take as input the sequence and to output a segmentation of the organ into characteristic portions (*e.g.* ventricle and atrium for the heart); or
- tracking of the organ based on a sequence of ultrasound images of the organ, *i.e.* the neural network is trained to take as input the sequence and to output the transformation (motion field) to geometrically align the organ along the sequence; or
- detection of the organ based on a sequence of ultrasound images of the organ, *i.e.* the neural network is trained to take as input the sequence and to output the bounding boxes corresponding to the position of the organ for each frame of the sequence; or
- registration task based on a sequence of ultrasound images of the organ, *i.e.* the neural network is trained to take as input the sequence and the corresponding sequence from another view (or from another modality) and to output the transformation to geometrically align the organs; or
- classification task based on a sequence of ultrasound images of the organ, *i.e.* the neural network is trained to take as input the sequence and to output classification of the organs or their characteristics (*e.g.* benign or malignant).

Neural networks for these tasks are well-known, as well as their learning. The present disclosure however brings new training data, the long sequences generated of ultrasound images, to improve the quality of the learning.

It is also proposed a neural network obtainable according to the method of use, *i.e.* a neural network having the weights and parameters equal to the weights/parameters resulting from the training according to the method of use, for example a neural network directly resulting from the training according to the method of use, having its weights and

parameters directly set by training according to the method of use. It is also proposed a computer-implemented method of use of this neural network in inference, to perform one of the above task on a real-world measured (by appropriate medical measurement device) sequence of ultrasound images of the organ. The method of use of the neural network thus comprises:

- obtaining a measured long sequence of ultrasound images of the organ, for example by actually performing the ultrasound imaging process or by simply obtaining an already acquired sequence; and
- applying the neural network to the obtained sequence to perform the task for which the neural network has been trained.

The method of use of the neural network may be performed independently from the other methods herein, or they may alternatively be performed in the same computer-implemented process.

The methods of the present disclosure are computer-implemented. This means that steps (or substantially all the steps) of the methods are executed by at least one computer, or any system alike. Thus, steps of the methods are performed by the computer, possibly fully automatically, or, semi-automatically. In examples, the triggering of at least some of the steps of the methods may be performed through user-computer interaction. The level of user-computer interaction required may depend on the level of automatism foreseen and put in balance with the need to implement user's wishes. In examples, this level may be user-defined and/or predefined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

FIG. 12 shows an example of the system, wherein the system is a client computer system, *e.g.* a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer-implemented method for generation of a sequence of synthetic ultrasound images of an organ, the method comprising:

   - providing:

     o a trained video diffusion model, the video diffusion model being trained to take as input a sequence of 2D representations of the organ each labelled with semantic labels and a corresponding sequence of noise images, and to generate a corresponding denoised sequence of synthetic ultrasound images of the organ respecting the semantic labels; and
     o several consecutive sequences of 2D representations of the organ each labelled with semantic labels and corresponding consecutive sequences of noise images; and

   - iteratively applying the video diffusion model to each couple formed by one consecutive sequence of 2D representations of the organ labelled with semantic labels and one corresponding sequence of noise images, including, at each iteration, when applying the video diffusion model to a given couple, using, for each denoising step of the backward process of the video diffusion model, a part of the denoised synthetic ultrasound images generated at the previous iteration for that denoising step in replacement of a part of images of the given couple for that denoising step.

2. The method of claim 1, wherein said part of the denoised synthetic ultrasound images generated at the previous iteration consists in ending denoised synthetic ultrasound images generated at the previous iteration and said part of images of the given couple consists in beginning images.

3. The method of claim 1 or 2, wherein a total number of images in the several consecutive sequences is larger than 40 images, and the number of images in each sequence is smaller than or equal to 24.

4. The method of claim 3, wherein the number of images in each sequence equals 16.

5. The method of claim 3 or 4, wherein the number of images in said part of the denoised synthetic ultrasound images generated at the previous iteration consists in ending denoised synthetic ultrasound images generated at the previous iteration and of images in said part of images of the given couple consists in beginning images is comprised between a quarter of and half of the number of images in each sequence.

6. The method of any one of claims 1 to 5, wherein the organ is a heart, a liver, a kidney, a carotid, an aorta, or a coronary.

7. A database comprising sequences of synthetic ultrasound images of an organ generated according to the method of any one of claims 1 to 6.

8. A computer-implemented method of use of the database of claim 7, comprising training a neural network based on the database, the neural network being trained for:

   - segmentation of the organ based on a sequence of ultrasound images of the organ; or
   - tracking of the organ based on a sequence of ultrasound images of the organ; or
   - detection of the organ based on a sequence of ultrasound images of the organ; or
   - registration task based on a sequence of ultrasound images of the organ; or
   - classification task based on a sequence of ultrasound images of the organ.

9. A neural network obtainable according to the method of claim 8.

10. A computer program comprising instructions which, when executed by a computer system, cause the system to perform the method of any one of claims 1 to 6 and/or the method of claim 8.

11. A computer-readable data storage medium having recorded thereon the computer program of claim 10 and/or the database of claim 7 and/or the neural network of claim 9.

12. A computer system comprising a processor coupled to a memory, the memory having recorded thereon the computer

program of claim 10 and/or the database of claim 7 and/or the neural network of claim 9.

**Amended claims in accordance with Rule 137(2) EPC.**

1. A computer-implemented method for generation of a sequence of synthetic ultrasound images of an organ, the method comprising:

   - providing:

     ◦ a trained video diffusion model, the video diffusion model being trained to take as input a sequence of 2D representations of the organ each labelled with semantic labels and a corresponding sequence of noise images, and to generate a corresponding denoised sequence of synthetic ultrasound images of the organ respecting the semantic labels; and
     o several consecutive sequences of 2D representations of the organ each labelled with semantic labels and corresponding consecutive sequences of noise images; and

   - iteratively applying the video diffusion model to each couple formed by one consecutive sequence of 2D representations of the organ labelled with semantic labels and one corresponding sequence of noise images, including, at each iteration, when applying the video diffusion model to a given couple, using, for each denoising step of the backward process of the video diffusion model, a part of the denoised synthetic ultrasound images generated at the previous iteration for that denoising step in replacement of a part of images of the given couple for that denoising step,

   wherein said part of the denoised synthetic ultrasound images generated at the previous iteration consists in ending denoised synthetic ultrasound images generated at the previous iteration and said part of images of the given couple consists in beginning images.

2. The method of claim 1, wherein a total number of images in the several consecutive sequences is larger than 40 images, and the number of images in each sequence is smaller than or equal to 24.

3. The method of claim 2, wherein the number of images in each sequence equals 16.

4. The method of claim 2 or 3, wherein the number of images in said part of the denoised synthetic ultrasound images generated at the previous iteration consists in ending denoised synthetic ultrasound images generated at the previous iteration and of images in said part of images of the given couple consists in beginning images is comprised between a quarter of and half of the number of images in each sequence.

5. The method of any one of claims 1 to 4, wherein the organ is a heart, a liver, a kidney, a carotid, an aorta, or a coronary.

6. A database comprising sequences of synthetic ultrasound images of an organ generated according to the method of any one of claims 1 to 5.

7. A computer-implemented method of use of the database of claim 6, comprising training a neural network based on the database, the neural network being trained for:

   - segmentation of the organ based on a sequence of ultrasound images of the organ; or
   - tracking of the organ based on a sequence of ultrasound images of the organ;
   or
   - detection of the organ based on a sequence of ultrasound images of the organ; or
   - registration task based on a sequence of ultrasound images of the organ; or
   - classification task based on a sequence of ultrasound images of the organ.

8. A neural network obtainable according to the method of claim 7.

9. A computer program comprising instructions which, when executed by a computer system, cause the system to perform the method of any one of claims 1 to 5 and/or the method of claim 7.

10. A computer-readable data storage medium having recorded thereon the computer program of claim 9 and/or the

neural network of claim 8.

**11.** A computer system comprising a processor coupled to a memory, the memory having recorded thereon the computer program of claim 9 and/or the database of claim 6 and/or the neural network of claim 8.

Forward process (fixed)
$s_T$
...
$s_t$
$p_\theta(s_{t-1}|s_t)$
$s_{t-1}$
...
$s_0$
$q(s_t|s_{t-1})$
$q(s_{t-1}|s_t)$ is unknown
Backward process

FIG. 1

Encoder
Decoder
Input Conv
Semantic Diffusion Encoder Resblock
Downsample
Semantic Diffusion Encoder Resblock
Spatial Attention
Temporal Attention
Semantic Diffusion Decoder Resblock
Spatial Attention
Temporal Attention
Semantic Diffusion Decoder Resblock
Semantic Diffusion Decoder Resblock
Spatial Attention
Temporal Attention
Upsample
Semantic Diffusion Decoder Resblock
Output Conv

FIG. 2

Variational
autoencoder
x
ε
D
x̃
Diffusion process
z
$z_T$
z
...
$z_{T-1}$
Denoising Unet
$z_T$
Pixel Space
Latent Space
Latent Diffusion Model

FIG. 3

1st 16 frames Generation
2nd 16 frames Generation
$\mathcal{Y}_0$
$\mathcal{Y}_1$
$S_0^T$
$S_0^{T-1}$
$S_0^1$
$S_0^0$
$S_1^T$
$S_1^{T-1}$
$S_1^1$
$S_1^0$

FIG. 4

s
f
h
w
f: number of frames
s: number of denoising steps
h: height
w: width

FIG. 5

Sequence-based conditioning
y-t slice
t
video
Noise blending
y-t slice
t
video

FIG. 6

dice latent
dice pixel
iou latent
iou pixel
c=1; lv blood pool
c=2; lv myocardium
c=2; atrium blood pool

FIG. 7

FIG. 8

FIG. 9

FIG. 10

① adventitia
② lumen
③ catheter region
④ calcification
⑤ soft plaque
⑥ shadow artefact
⑦ guidewire artefact

FIG. 11

1000
1010
CPU
1070
RAM
1020
Mass storage
devices controler
Display
1080
B
U
S
1030
Hard
drive
Haptic
device
1090
Network Adapter
Video
RAM
1050
1100
Network
GPU
1110
1060

FIG. 12

Europäisches
Patentamt
European
Patent Office
Office européen
des brevets

# EUROPEAN SEARCH REPORT

Application Number
EP 25 30 5198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br>A | PHI NGUYEN VAN ET AL: "Echocardiography video synthesis from end diastolic semantic map via diffusion model", ARXIV.ORG, CORNELL UNIVERSITY LIBRARY, 201 OLIN LIBRARY CORNELL UNIVERSITY ITHACA, NY 14853, 11 October 2023 (2023-10-11), XP091632764, * page 2 - page 3 * ----- | 1,3,4, 6-12<br><br><br><br><br><br><br>2,5 | INV.<br>G06T5/60<br>G06T5/70 |
| A | STOJANOVSKI DAVID ET AL: "Echo from Noise: Synthetic Ultrasound Image Generation Using Diffusion Models for Real Image Segmentation", 1 October 2023 (2023-10-01), SIMPLIFYING MEDICAL ULTRASOUND; [LECTURE NOTES IN COMPUTER SCIENCE; LECT.NOTES COMPUTER], SPRINGER NATURE SWITZERLAND, CHAM, PAGE(S) 34 - 43, XP047671720, ISSN: 0302-9743 ISBN: 978-3-031-44520-0 [retrieved on 2023-10-01] * the whole document * ----- | 1-12 | TECHNICAL FIELDS SEARCHED (IPC)<br>G06T |
| A | SUTHAR HARSH ET AL: "Synthesizing Ultrasound Datasets using Mask Conditional Diffusion Models", 2024 IEEE ULTRASONICS, FERROELECTRICS, AND FREQUENCY CONTROL JOINT SYMPOSIUM (UFFC-JS), IEEE, 22 September 2024 (2024-09-22), pages 1-5, XP034781621, DOI: 10.1109/UFFC-JS60046.2024.10793641 [retrieved on 2024-12-18] * the whole document * ----- | 1-12 | |

The present search report has been drawn up for all claims

2

| Place of search | Date of completion of the search | Examiner |
|---|---|---|
| Munich | 2 June 2025 | Üreyen, Soner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Non-patent literature cited in the description


- **WANG et al.** Semantic image synthesis via diffusion models.. *arXiv preprint arXiv:2207.00050*, 2022
- **HO et al.** Video Diffusion Models. *NeurIPS*, 2022
- **ISENSEE**. nnu-net: Self-adapting framework for u-net-based medical image segmentation.. *arXiv preprint arXiv:1809.10486*, 2018
- **MARQUEZ-NEILA et al.** A morphological approach to curvature-based evolution of curves and surfaces. *IEEE Transactions on Pattern Analysis and Machine Intelligence*, 2013, vol. 36 (1), 2-17
- **OHTE, N** ; **ISHIZU, T** ; **IZUMI, C** ; **ITOH, H** ; **IWANAGA, S** ; **OKURA, H** ; **JAPANESE CIRCULATION SOCIETY JOINT WORKING GROUP.** JCS 2021 guideline on the clinical application of echocardiography. *Circulation Journal*, 2022, vol. 86 (12), 2045-2119
- **TOWNSEND, R. R.** ; **WILKINSON, I. B.** ; **SCHIFFRIN, E. L** ; **AVOLIO, A. P.** ; **CHIRINOS, J. A** ; **COCKCROFT, J. R.** ; **WEBER, T.** Recommendations for improving and standardizing vascular research on arterial stiffness: a scientific statement from the American Heart Association.. *Hypertension*, 2015, vol. 66 (3), 698-722
- **ISSELBACHER, E. M** ; **PREVENTZA, O** ; **HAMILTON BLACK III** ; **J., AUGOUSTIDES** ; **J. G.** ; **BECK, A. W** ; **WOO, Y. J.** ACC/AHA guideline for the diagnosis and management of aortic disease: a report of the American Heart Association/American College of Cardiology Joint Committee on Clinical Practice Guidelines. *Journal of the American College of Cardiology*, 2022, vol. 80 (24), e223-e393
- **KELLY, E. M** ; **FELDSTEIN, V. A** ; **PARKS, M.** ; **HUDOCK, R** ; **ETHERIDGE, D** ; **PETERS, M. G**. An assessment of the clinical accuracy of ultrasound in diagnosing cirrhosis in the absence of portal hypertension.. *Gastroenterology & hepatology*, 2018, vol. 14 (6), 367
- **HANSEN, K. L** ; **NIELSEN, M. B.** ; **EWERTSEN, C.** Ultrasonography of the kidney: a pictorial review.. *Diagnostics*, 2015, vol. 6 (1), 2
- **XIA, M** ; **YAN, W** ; **HUANG, Y** ; **GUO, Y.** ; **ZHOU, G** ; **WANG, Y.** IVUS Image Segmentation Using Superpixel-Wise Fuzzy Clustering and Level Set Evolution.. *Appl. Sci.*, 2019, vol. 9, 4967, https://doi.org/10.3390/app9224967
- **RIZI, F. Y.** ; **AU, J** ; **YLI-OLLILA, H** ; **GOLEMATI, S** ; **MAKUNAITÉ, M.** ; **ORKISZ, M** ; **ZAHND, G.** Carotid wall longitudinal motion in ultrasound imaging: an expert consensus review. *Ultrasound in Medicine & Biology*, 2020, vol. 46 (10), 2605-2624